(19) **Europäisches Patentamt**

**European Patent Office**

**Office européen des brevets**

(11) Numéro de publication : **0 434 628 B1**

(12) # FASCICULE DE BREVET EUROPEEN

(45) Date de publication du fascicule du brevet :
02.03.94 Bulletin 94/09

(21) Numéro de dépôt : **90810992.9**

(22) Date de dépôt : **17.12.90**

(51) Int. Cl.⁵ : **A61K 7/48,** A61K 33/40, A61K 31/07

(54) Composition à usage cosmétique ou pharmaceutique.

(30) Priorité : **21.12.89 CH 4582/89**

(43) Date de publication de la demande :
**26.06.91 Bulletin 91/26**

(45) Mention de la délivrance du brevet :
**02.03.94 Bulletin 94/09**

(84) Etats contractants désignés :
**AT BE CH DE DK ES FR GB GR IT LI LU NL SE**

(56) Documents cités :
**CH-A- 670 951**

(73) Titulaire : **Herzog- Thomander, Karin**
**37-39 Route du Monteliza**
**CH-1806 St-Légier (CH)**

(72) Inventeur : **Hezog, Paul**
**37-39, Route du Monteliza**
**CH-1806 Saint-Légier (CH)**

(74) Mandataire : **Vuille, Roman**
**c/o Laboratoire de Recherches Docteur méd.**
**et Mme Paul Herzog Route du Montéliza 37-39**
**CH-1806 St-Légier (CH)**

EP 0 434 628 B1

## Description

La présente invention a pour objet une nouvelle composition à usage cosmétique ou pharmaceutique. Exclusivement destinée à un usage externe, elle permet de traiter efficacement diverses affections de la peau.

L'action thérapeutique de la vitamine A sous sa forme acide (acide rétinoïque), aldéhyde (rétinal) ou alcool (rétinol) est bien connue en dermatologie.

L'utilisation d'une composition utilisant conjointement un ester de vitamine A avec une émulsion stable de peroxyde d'hydrogène a été proposée dans le brevet CH-A-670.951. Une telle émulsion possède un pouvoir libérateur d'oxygène particulièrement élevé (pression paraosmotique de près de 10 atm), ce qui a notamment pour effet d'accélérer le passage du dérivé de vitamine A au travers des couches superficielles de la peau où la conversion de l'ester de vitamine A en acide rétinoïque peut avoir lieu.

Katzberg, Anat. Rec. **112**, 418 (1952), puis H. Pinkus, Dermatologica, **106**, 28 (1953) ont trouvé que le temps de vie d'une cellule épidermique humaine est de 101 jours dans les premiers dix ans et diminue jusqu'à 46 jours à l'âge de 80 ans. Cela signifie que le temps occasionne un vieillissement qui se manifeste par une diminution de l'aptitude des cellules à régénérer l'épiderme. Quand l'énergie physiologiquement utilisable par les cellules n'est plus suffisante, par exemple par suite d'une insuffisance de perméabilité des capillaires ou de circulation capillaire, il y a une accélération de la disparition des cellules avec une forte atteinte à leurs fonctions organiques.

Si la multiplication et la croissance des cellules somatiques épidermiques nécessite de l'oxygène, la vie de la cellule dépend de la glycolyse qui est une dégradation oxydative du glucose. En effet, pour la conservation de sa structure, pour être prête à fonctionner ou pour exercer ses fonctions, chaque cellule individuelle de l'organisme a besoin d'une grande quantité d'énergie.

Si l'énergie permettant le fonctionnement de la cellule manque même partiellement, cela peut conduire à des dégâts qui sont réversibles au début mais qui, s'ils ne sont pas traités, mènent à la fin du maintien de la structure de la cellule et par la suite entraînent sa mort prématurée.

Pour l'instant, on ne dispose pas de composition à usage cosmétique ou pharmaceutique associant les effets de la vitamine A et du peroxyde d'hydrogène et permettant en outre de fournir l'énergie dont la cellule a besoin.

La nouvelle composition selon l'invention fournit cette énergie et permet de ce fait un rétablissement immédiat des fonctions de chaque cellule individuelle si l'insuffisance capillaire est due à un manque d'approvisionnement en énergie, ce qui est particulièrement bénéfique, au niveau des tissus cutanés.

L'invention a plus précisément pour objet une composition à usage cosmétique ou pharmaceutique contenant de la vitamine A sous forme d'ester ou d'acide et du glucose en association avec une émulsion aqueuse stable de peroxyde d'hydrogène.

L'énergie est fournie par la dégradation oxydative du glucose qui est rendue possible grâce à l'offre suffisante d'oxygène due au peroxyde d'hydrogène.

Le glucose est transporté au travers des couches superficielle de la peau avec la vitamine A (ester ou acide libre) grâce à la pression de l'oxygène naissant. Sous la peau, l'exploitation de l'énergie libérée par la glycolyse peut ainsi avoir lieu.

Dans des conditions in-vivo, la dégradation oxydative de 1 mole de glucose ($C_6H_{12}O_6 + 6O_2 = 6CO_2 + 6H_2O$) conduit à la formation de 690 kcal et est liée à la formation de 38 moles d'adénosine triphosphate (ATP).

La glycolyse est plus précisément une dégradation oxydative du glucose dans un organisme vivant sous l'effet d'enzymes. L'ATP sert de donneur de phosphate riche en énergie pour de nombreuses réactions de phosphorylation, et sert aussi à la synthèse des acides ribonucléinques.

L'application de la composition selon l'invention, une crème par exemple, permet de maintenir une décomposition rapide de l'ATP, dans les tissus cutanés au contact de ladite composition, en adénosine diphosphate (ADP) qui libère l'énergie indispensable pour le métabolisme énergétique dans la cellule: la synthèse du collagène, facteur important du maintien de l'élasticité du derme notamment.

Les émulsions de peroxyde d'hydrogène utilisables dans le cadre de la présente invention peuvent être du type eau-dans-huile aussi bien que huile-dans-eau.

Une émulsion de peroxyde d'hydrogène huile-dans-eau stable, particulièrement bien adaptée à la préparation de compositions selon l'invention est décrite dans le brevet US-A-3.954.974.

Etant donné l'absence de produits secondaires toxiques lors de la décomposition du peroxyde d'hydrogène, de telles émulsions peuvent présenter une concentration élevée en oxygène naissant qui renforce l'autant l'action de la vitamine A tout en permettant la libération d'énergie par suite de sa réaction avec le glucose.

A titre d'ester de vitamine A, on utilise de préférence un ester d'acide gras tel que le palmitate de vitamine A, ou encore l'acétate correspondant. De tels produits se trouvent dans le commerce sous une forme appropriée; les esters d'acide gras de vitamine A ont en outre l'avantage d'être bien tolérés par la peau et l'organisme.

Les concentrations relatives en ester ou en acide de vitamine A et de glucose peuvent varier fortement en fonction des effets recherchés.Pour ce qui est du dérivé de vitamine A, les concentrations avantageusement applicables varient de 1'000 à 10'000 Unités Internationales (U.I.: exprimées en vitamine A) par gramme de composition selon l'invention

Pour ce qui est du glucose, il convient d'incorporer au sein de la composition la fraction molaire adéquate procurant, par glycolyse, la quantité d'énergie requise par le métabolisme cellulaire. On utilise avantageusement le glucose à raison de 0,5 à 10‰ en poids de glucose par rapport au poids de composition.

Les concentrations de peroxyde d'hydrogène dans la composition varient évidemment selon l'effet oxygénant recherché. Dans le cas de compositions à usage cosmétique, on utilise le peroxyde d'hydrogène à raison de 0.5 à 4% en poids (valeur exprimée en $H_2O_2$ 100%), dans le cas de compositions à usage pharmaceutique de 0,1 à 10% en poids voire plus selon les cas, par rapport au poids total de la composition.

Par la composition équilibrée de l'émulsion, il est assuré que dans les tissus cutanés l'excès d'oxygène produit non seulement une dégradation oxydative immédiate du glucose, mais en outre que le palmitate ou l'acétate de vitamine A est transformé en acide rétinoïque, et que le milieu extracellulaire est enrichi en oxygène et en eau, produits résultant tous deux de la décomposition du peroxyde d'hydrogène.

Les compositions selon l'invention peuvent bien entendu contenir les ingrédients stabilisants, épaississants, parfumants ou colorants usuels. Elles peuvent également contenir des ingrédients actifs additionnels comme par exemple d'autres vitamines ou dérivés de vitamines, tels que la vitamine E ou des liposomes.

Etant à base d'émulsion aqueuse à pouvoir oxygénant élevé, les compositions selon l'invention présentent en outre un effet désinfectant et cicatrisant fort avantageux et se révèlent utiles pour le traitement de brûlures, de blessures ouvertes telles qu'ulcères ou les troubles consécutifs aux hémorroïdes.

Les exemples ci-après sont destinés à illustrer l'invention de manière plus détaillée. Ces exemples ne sont en aucun cas limitatifs.

## Exemple 1 - Composition cosmétique

On prépare une première phase de type "huile" en mélangeant les ingrédients suivants :

```
Vaseline                450 g
Paraffine liquide       325 g
Alcool cétylique        160 g
Alcool stéarylique      160 g
Monostéarine            310 g
                        ————
Total                  1405 g
```

On effectue séparément le mélange des constituants suivants pour obtenir une phase de type "eau" :

| | | |
|---|---|---|
| H$_2$O$_2$ 30% | 400 g = | 0,353 mol/kg de crème |
| "Tween 80" | 125 g | |
| Acide salicylique | 9 g | |
| Palmitate de vitamine A* | 63 g | |
| Acétate de D,L-α-tocophérol | 200 g | |
| Glucose | 18 g = | 0,01 mol/kg de crème |
| Eau distillée | 7780 g | |
| Total | 8595 g | |

\* 1,7 mio UI/g

Les phases "huile" et "eau" ainsi préparées sont ensuite mélangées à une température de 70 à 80°C dans un appareillage adéquat, jusqu'à l'obtention d'une émulsion homogène. Ceci conduit à 10 kg de crème à usage cosmétique contenant 1,1% d'oxygène actif, 0,01 mol/kg de glucose et 10'000 UI de palmitate de vitamine A/g de crème.

**Exemple 2 - Composition cosmétique**

On prépare une première phase de type "huile" en mélangeant les ingrédients suivants :

| | |
|---|---|
| Vaseline | 450 g |
| Paraffine liquide | 325 g |
| Alcool cétylique | 160 g |
| Alcool stéarylique | 160 g |
| Monostéarine | 310 g |
| Palmitate de vitamine A* | 63 g |
| Acétate de D,L-α-tocophérol | 200 g |
| Total | 1668 g |

\* 1,7 mio UI/g

On effectue séparément le mélange des constituants suivants pour obtenir une phase de type "eau" :

| H₂O₂ 30 % | 1167 g = 1,03 mol/kg de crème |
|---|---|
| "Tween 80" | 125 g |
| Acide salicylique | 9 g |
| Glucose | 18 g = 0,01 mol/kg de crème |
| Eau distillée | 7013 g |
| Total | 8332 g |

Les phases "huile" et "eau" ainsi préparées sont ensuite mélangées à une température de 70 à 80°C dans un appareillage adéquat, jusqu'à l'obtention d'une émulsion homogène. Ceci conduit à 10 kg de crème à usage cosmétique contenant 3,5% d'oxygène actif, 0,01 mol/kg de glucose et 10'000 UI de palmitate de vitamine A/g de crème.

**Exemple 3 - Composition pharmaceutique**

On prépare une première phase de type "huile" en mélangeant les ingrédients suivants :

| Vaseline | 475 g |
|---|---|
| Paraffine liquide | 350 g |
| Alcool cétyliaue | 175 g |
| Alcool stéarylique | 175 g |
| Monostéarine | 350 g |
| D,L-α-tocophérol Vit. E | 250 g |
| Total | 1775 g |

On effectue séparément le mélange des constituants suivants pour obtenir une phase de type "eau" :

| H₂O₂ 30% | 1176 g |
|---|---|
| "Tween 80" | 150 g |
| Acide salicylique | 11 g |
| Glucose | 18 g |
| Acide rétinoïque "Trétinoïne" | 5 g |
| Eau distillée | 6865 g |
| Total | 8225 g |

Les phases "huile" et "eau" ainsi préparées sont ensuite mélangées à une température de 70 à 80°C dans un appareillage adéquat, jusqu'à l'obtention d'une émulsion homogène. Ceci conduit à 10 kg de crème à usage pharmaceutique contenant 3,5% d'oxygène actif (peroxyde d'hydrogène), 0,01 mol/kg de glucose et 0.05% d'acide rétinoïque ("Trétinoïne").

Ces compositions conviennent parfaitement bien à de nombreux usages cosmétiques ou pharmaceutiques et se sont révélées particulièrement efficaces dans les applications cosmétiques visant à lutter contre le vieillissement de la peau. Elles peuvent être également utilisées dans le traitement d'affections bénignes de la peau telles que l'acné. Ces compositions possèdent en outre un effet désinfectant.

## Revendications

1. Composition à usage cosmétique contenant de la vitamine A sous forme d'ester ou d'acide libre et du glucose, en association avec une émulsion aqueuse stable de peroxyde d'hydrogène.

2. Composition à usage pharmaceutique contenant de la vitamine A sous forme d'ester ou d'acide libre et du glucose, en association avec une émulsion aqueuse stable de peroxyde d'hydrogène.

3. Composition selon l'une des revendication 1 et 2, caractérisée en ce que l'ester de vitamine A est choisi parmi le palmitate et l'acétate.

4. Composition selon l'une des revendications 1 à 3, caractérisée en ce que la vitamine A, sous forme d'acide libre ou d'ester, est présente à raison de 1'000 à 10'000 UI/g de composition.

5. Composition selon l'une des revendications 1 à 4, caractérisée en ce que le glucose est présent à raison de 0,5 à 10‰ en poids par rapport au poids de composition.

6. Composition selon l'une des revendications 1 à 5, caractérisée en ce que l'émulsion aqueuse stable de peroxyde d'hydrogène est une émulsion de type huile-dans-eau.

7. Composition à usage cosmétique selon l'une des revendications 1 et 3 à 6, caractérisée en ce qu'elle contient de 0,5 à 4% en poids de peroxyde d'hydrogène par rapport au poids de composition.

8. Composition à usage pharmaceutique selon l'une des revendications 2 à 6, caractérisée en ce qu'elle contient de 0,1 à 10% en poids de peroxyde d'hydrogène par rapport au poids de composition.

## Patentansprüche

1. Mittel zur kosmetischen Verwendung, welches Vitamin A als Ester oder freie Saüre und Glucose, mit einer stabilen wässrigen Wasserstoffperoxyd Emulsion kombiniert, enthält.

2. Mittel zur pharmazeutischen Verwendung, welches Vitamin A als Ester oder freie Saüre und Glucose, mit einer stabilen wässrigen Wasserstoffperoxyd Emusion kombiniert, enthält.

3. Mittel nach einem der Ansprüche 1 und 2, dadurch gekennzeichnet, dass das Vitamin A Ester aus dem Palmitat und dem Essigsaüreester gewählt wird.

4. Mittel nach einem der Ansprüche 1 bis 3, dadurch gekennzeichnet, dass Vitamin A, als freie Saüre oder als Ester, in Mengen von 1'000 bis 10'000 IE/g Mittel gegenwärtig ist.

5. Mittel nach einem der Ansprüche 1 bis 4, dadurch gekennzeichnet, dass Glucose in Mengen von 0,5 bis 10 Gew.-o/oo pro Mittels Gewicht gegenwärtig ist.

6. Mittel nach einem der Ansprüche 1 bis 5, dadurch gekennzeichnet, dass die stabile wässrige Wasserstoffperoxyd Emulsion eine Oel-in-Wasser Emulsion ist.

7. Mittel zur kosmetischen Verwendung nach einem der Ansprüche 1 und 3 bis 6, welches von 0,5 bis 4 Gew.-% Wasserstoffperoxyd pro Mittels Gewicht enthält.

8. Mittel zur pharmazeutischen Verwendung nach einem der Ansprüche 2 bis 6, welches von 0,1 bis 10 Gew.-% Wasserstoffperoxyd pro Mittels Gewicht enthält.

**Claims**

1. Composition for cosmetic use comprising vitamin A as an ester or as free acid and glucose in combination with a stable aqueous emulsion of hydrogen peroxide.

2. Composition for pharmaceutical use comprising vitamin A as an ester or as free acid and glucose in combination with a stable aqueous emulsion of hydrogen peroxide.

3. Composition according to one of claims 1 and 2, wherein the vitamin A ester is selected from the palmitate and the acetate.

4. Composition according to one of claims 1 to 3, wherein vitamin A either as an ester or as free acid is present in amounts of 1'000 to 10'000 IU/g of composition.

5. Composition according to one of claims 1 to 4, wherein glucose is present in amounts of 0.5 to 10 o/oo by weight with respect to the weight of the composition.

6. Composition according to one of claims 1 to 5, wherein the stable aqueous hydrogen peroxide emulsion is an oil-in-water emulsion.

7. Composition for cosmetic use according to one of claims 1 and 3 to 6 comprising from 0.5 to 4 % weight of hydrogen peroxide per weight of composition.

8. Composition for pharmaceutical use according to one of claims 2 to 6 comprising from 0.1 to 10 % weight of hydrogen peroxide per weight of composition.